# EUROPEAN PATENT SPECIFICATION

(11) **EP 3 042 696 B1**
(45) Date of publication and mention of the grant of the patent: **28.06.2017**
(21) Application number: 14841661.3
(22) Date of filing: 30.07.2014
(51) Int. Cl.: A61N 5/06

(54) **LIGHT EMITTING DEVICE FOR BODY HAIR**
LICHTEMITTIERENDE VORRICHTUNG FÜR KÖRPERHAARE
DISPOSITIF ÉMETTANT DE LA LUMIÈRE POUR POILS CORPORELS

(30) Priority: 06.09.2013 JP 2013185161
(43) Date of publication of application: 13.07.2016
(73) Proprietor: Panasonic Intellectual Property Management Co., Ltd., Osaka-shi, Osaka 540-6207 (JP)
(72) Inventor: NAKAGAWA, Takehiro, Osaka-shi Osaka 540-6207 (JP); FUJIKAWA, Shoji, Osaka-shi Osaka 540-6207 (JP)
(74) Representative: Grünecker Patent- und Rechtsanwälte PartG mbB
(86) International application number: PCT/JP2014/004002
(87) International publication number: WO 2015/033513

(56) References cited:
- EP-A1- 2 260 901
- WO-A1-2009/111010
- WO-A1-2011/034121
- DE-A1-102007 007 777
- JP-A- 2011 125 739
- US-A1- 2007 198 004
- US-A1- 2010 100 159

## Description

### TECHNICAL FIELD

The present invention relates to a light emitting device for body hair

### BACKGROUND ART

Patent document 1 discbses a body hair light emitting device that includes an LED lamp. The LED lamp emits light having a peak in the unique absorption wavelength of water in the near-infrared region. Patent document 1 discloses that when such light is emitted to a living body, the light acts to promote the hair growth effect of the living body.

### PRIOR ART DOCUMENT

### PATENT DOCUMENT

Patent Document 1: WO 2009/123196

### SUMMARY

### PROBLEMS TO BE SOLVED BY THE INVENTION

The inventors of the present invention have noticed that the above body hair light emitting device has a shortcoming in that the hair growth effect becomes difficult to promote as the light emission time becomes longer.

It is an object of the present invention to provide a body hair light emitting device that limits decreases in the amount of light absorbed by the living body even when the emission time of light increases.

### MEANS FOR SOLVING THE PROBLEM

In one aspect, a body hair light emitting device includes a light source that outputs light and a controller that controls the output of the light source. The controller is connected to the light source. The controller controls the output of the light source so that output light of the body hair light emitting device resulting from the output of the light source has a main peak region including an output light peak, which indicates maximum intensity, in a wavelength range from 1350 nm to 1550 nm and so that an integral value of a peak region short-wavelength component, which is a main peak region component at a short-wavelength side of the output light peak, is larger than an integral value of a peak region long-wavelength component, which is a main peak region component at a long-wavelength side of the output light peak.

When the above integral value relationship is established, the decrease in the amount of light absorbed by a living body is limited even if the emission time of light increases compared to when the integral value of the peak region short-wavelength component is less than or equal to the integral value of the peak region long-wavelength component.

### EFFECT OF THE INVENTION

The hair body light emitting device of the above embodiment limits decreases in the amount of light absorbed by the living body even when the emission time of light increases,

### BRIEF DESCRIPTION OF THE DRAWINGS

Fig. 1 is a block diagram of one embodiment of a body hair light emitting device in one embodiment;
Fig. 2 is a cross-sectional view of a light source unit illustrated in Fig. 1;
Fig. 3A is a graph illustrating the spectrum of light in one embodiment;
Fig. 3B is a graph illustrating the spectrum of light in one embodiment when the light source temperature rises;
Fig. 3C is a graph illustrating the spectrum of light in one embodiment when the light source temperature further rises;
Fig. 4A is a graph illustrating the spectrum of light in a comparative example;
Fig. 4B is a graph illustrating the spectrum of light in the comparative example when the light source temperature rises; and
Fig. 4C is a graph illustrating the spectrum of light in the comparative example when the light source temperature further rises.

### EMBODIMENTS OF THE INVENTION

A representative embodiment for the purpose of exemplification will now be described. The disclosure of the present specification is not intended to entirely cover the invention with only the representative embodiment. The disclosure also includes various embodiments obtained by at least partially deleting or replacing the structure of the embodiment or by adding other structures. The present disclosure basically includes the following embodiment.

In one embodiment, a body hair light emitting device includes a light source that outputs light and a controller that controls the output of the light source. The controller is connected to the light source. The controller controls the output of the light source so that output light of the body hair light emitting device resulting from the output of the light source has a main peak region including an output light peak, which indicates maximum intensity, in a wavelength range from 1350 nm to 1550 nm and so that an integral value of a peak region short-wavelength component, which is a main peak region component at a short-wavelength side of the output light peak, is larger than an integral value of a peak region long-wavelength component, which is a main peak region component at a long-wavelength side of the output light peak.

In one embodiment, preferably, a short-wavelength side maximum gradient, which is the maximum gradient of the main peak region at the short-wavelength side of the output light peak, is smaller than a long-wavelength side maximum gradient, which is the maximum gradient of the main peak region at the long-wavelength side of the output light peak.

In one embodiment, a light absorption spectrum of water has a light absorption peak region including a light absorption peak, which indicates maximum absorbance, in a wavelength range from 1440 nm to 1460 nm. Preferably, the controller controls spectrum of the output light relative to the light absorption spectrum of water so that the longwavelength side maximum gradient of the main peak region is larger than a longwavelength side light absorbance gradient, which is the maximum gradient of the light absorption peak region at the long-wave!ength side of the light absorption peak.

In one embodiment, a light absorption spectrum of water has a light absorption peak region including a light absorption peak, which indicates maximum absorbance, in a wavelength range from 1440 nm to 1460 nm. Preferably, the controller controls spectrum of the output light relative to the light absorption spectrum of water so that the short-wavelength side maximum gradient of the main peak region is smaller than a short-wavelength side light absorbance gradient, which is the maximum gradient of the light absorption peak region at the short-wavelength side of the light absorption peak.

In one embodiment, preferably, the output light does not include a significant peak region except for the main peak region in a wavelength range from 1200 nm to 1600 nm.

In one embodiment, the main peak region includes a short-wavelength base point, which is a base point of the main peak region existing at the short-wavelength side of the output light peak, and a long-wavelength base point, which is a base point of the main peak region existing at the long-wavelength side of the output light peak. Preferably, a line connecting the two points of the long-wavelength base point and the output light peak has a larger gradient than a line connecting the two points of the short-wavelength base point and the output light peak.

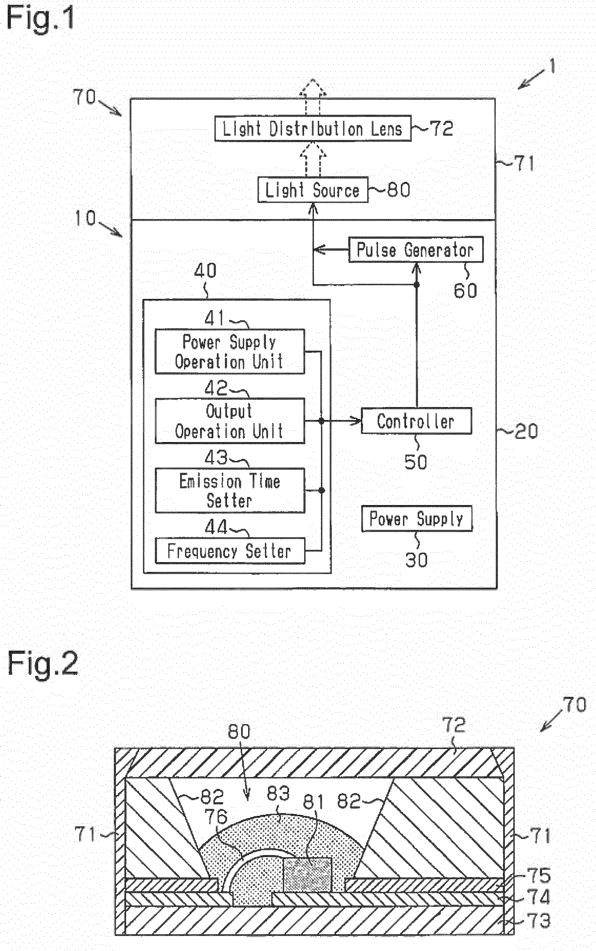

Fig. 1 is a block diagram illustrating one embodiment of a body hair light emitting device 1.

The body hair light emitting device 1 emits light to a living body to promote the hair growth effect. Hair growth is an action that promotes the reproduction and growth of body hair from a living body. The hair growth effect is an effect that promotes the growth of hair in the living body. The emission of light promotes the hair growth effect so that hair grows easily.

The body hair light emitting device 1 includes a main unit 10 and a light source unit 70. The main unit 10 includes a plurality of elements. The elements of the main unit 10 include, for example, a main unit housing 20, a power supply 30, an operation unit 40, and a pulse generator 60. The light source unit 70 includes a plurality of elements. The elements of the light source unit 70 include, for example, a light source housing 71, a light distribution lens 72, and a light source 80,

The body hair light emitting device 1 includes a plurality of electric blocks. The electric blocks are driven by power supplied from the power supply 30. The operation unit 40, the controller 50, the pulse generator 60, and the light source unit 70 each correspond to an electric block.

The main unit housing 20 is formed from, for example, a resin material. The main unit housing 20 is, for example, is formed to allow for hand-carrying. The main unit housing 20 has a hollow interior to accommodate the elements of the main unit 10,

The power supply 30 is located in the main unit housing 20. The power supply 30 is coupled to the main unit housing 20. Further, the power supply 30 is electrically connected to the operation unit 40, the controller 50, the pulse generator 60, and the light source unit 70. The power supply 30 supplies each electric block with power from a primary battery or a secondary battery.

The operation unit 40 is formed on the main unit housing 20. The operation unit 40 has, for example, a button-type form. The operation unit 40 includes a power supply operation unit 41, an output operation unit 42, an emission time setter 43, and a frequency setter 44.

The power supply operation unit 41 is electrically connected to the power supply 30. The power supply operation unit 41 can change operation positions when operated by an operator. When the operation position of the power supply operation unit 41 is an ON position, the power supply 30 supplies each electric block with power. When the operation position of the power supply operation unit 41 is an OFF position, the power supply 30 does not supply each electric block with power.

The output operation unit 42 is electrically connected to the controller 50. The output operation unit 42 outputs an operation signal ST to the controller 50 whenever operated by the operator. The operation signal ST includes information indicating that the output operation unit 42 has been operated.

The emission time setter 43 is electrically connected to the controller 50. The emission time setter 43 outputs a time setting signal SM whenever operated by the operator. The time setting signal SM includes information designating a specified time that is the time for outputting light from the light source 80. The content of the time setting signal SM changes in accordance with the number of times the emission time setter 43 is operated. The time setting signal SM designates the specified time of, for example, one of 5 minutes, 10 minutes, and 20 minutes.

The frequency setter 44 is electrically connected to the controller 50. The frequency setter 44 outputs a change request signal SC to the controller 50 whenever operated by the operator. The change request signal SC includes information designating the frequency of the light output from the light source 80 (hereinafter, "the output frequency TL"). The content of the change request signal SC changes in accordance with the number of times the frequency setter 44 is operated. The change request signal SC designates the output frequency TL of, for example, one of 0 Hz, 200 Hz, 500 Hz, and 1000 Hz. When the output frequency TL is 0 Hz, the light source 80 is driven in a continuous emission mode, which is a mode for continuously emitting light.

The light source housing 71 is formed from, for example, a resin material The light source housing 71 can be coupled to and separated from the main unit housing 20. The light source housing 71 has a hollow interior to accommodate the elements of the light source unit 70.

The light distribution lens 72 is formed from, for example, polymethylmethacrylate resin. The light distribution lens 72 has, for example, the shape of a truncated cone. The light distribution lens 72 is coupled to an opening of the light source housing 71,

The light source 80 is located in the light source housing 71. The light source 80 is coupled to the light source housing 71. Further, the light source 80 is electrically connected to the controller 50. The light source 80 outputs light in response to an output execution signal SA received from the controller 50. Further, the light source 80 stops the output of light in response to an output stop signal SB received from the controller 50. The light emitted from the light source 80 is output from the body hair light emitting device 1 through the light distribution lens 72. The light output from the body hair light emitting device 1 (hereinafter, "the output light") is emitted to a living body.

The pulse generator 60 is located in the main unit housing 20. The pulse generator 60 is electrically connected to the controller 50. In response to an output change signal SD received from the controller 50, the pulse generator 60 pulse-modulates the output execution signal SA, which is input to the light source 80. The pulse modulation of the output execution signal SA changes the frequency of the light output by the light source 80.

The controller 50 is located in the main unit housing 20, The controller 50 controls the operation of the light source unit 70 in accordance with the operation signal ST, the time setting signal SM, and the change request signal SC.

The controller 50 outputs the output execution signal SA or the output stop signal SB to the light source when receiving the operation signal ST. The controller 50 alternately outputs the output execution signal SA and the output stop signal SB, The output execution signal SA includes information for outputting light from the light source 80. The output stop signal SB includes information for stopping the output of light from the light source 80.

After starting the output of light from the light source 80, the controller 50 measures the time the light source 80 emits light When the measured time reaches a specified time, which is set in accordance with the time setting signal SM, the controller 50 outputs the output stop signal SB to the light source 80.

The controller 50 outputs the output change signal SD to the pulse generator when receiving the change request signal SC. The output change signal SD includes pulse modulation information corresponding to the information of the output frequency TL, which is included in the change request signal SC. The pulse modulation information pulse-modulates the output execution signal SA and changes the frequency of the light output from the light source 80. In this manner, the light source 80 outputs light in accordance with the instruction signal of the controller 50 (output execution signal SA, output stop signal SB. or output change signal SD).

Fig. 2 is a cross-sectional view illustrating one example of the light source unit 70.

The light source unit 70 includes the light source housing 71, the light distribution tens 72, and the light source 80. Additionally, the light source unit 70 includes a printed-circuit board 73, a conductive pattern 74, a resist 75. and a conductive component 76.

The printed-circuit board 73 is located in the light source housing 71. The printed-circuit board 73 is coupled to the light source housing 71. The printed-circuit board 73 is formed by, for example, a resin in which paper is impregnated with phenol. The conductive pattern 74 is formed on the printed-circuit board 73. The resist 75 is formed on the conductive pattern 74. The resist 75 exposes a portion of the conductive pattern 74. The conductive component 76 has the form of, for example, a wire. The conductive component 76 is connected to the conductive pattern 74.

The light source 80 has the form of, for example, an LED lamp. The light source 80 includes a plurality of elements. The elements of the light source 80 include an LED chip 81, a reflector 82, and a sealant 83.

The LED chip 81 is of a surface-mount type. The LED chip 81 is mounted on the printed-circuit board 73. The LED chip 81 is connected to the pattern 74 by the conductive component 76. The LED chip 81 is covered by the sealant 83.

The reflector 82 is located on the resist 75. The reflector 82 is attached to the resistor 75. The reflector 82 is formed from, for example, a metal material, a resin material, or a material having high reflectance. One example of a material having high reflectance is ceramics. The reflector 82 reflects light emitted from the LED chip 81 toward the light distribution lens 72.

The sealant 83 is arranged in a void defined by a reflection surface of the reflector 82. The sealant 83 is formed from, for example, a silicon resin, an epoxy resin, or a resin material having high transmittance. A resin material having a high transmittance is formed by a base material including glass or by mixing a light dispersant in a glass based material.

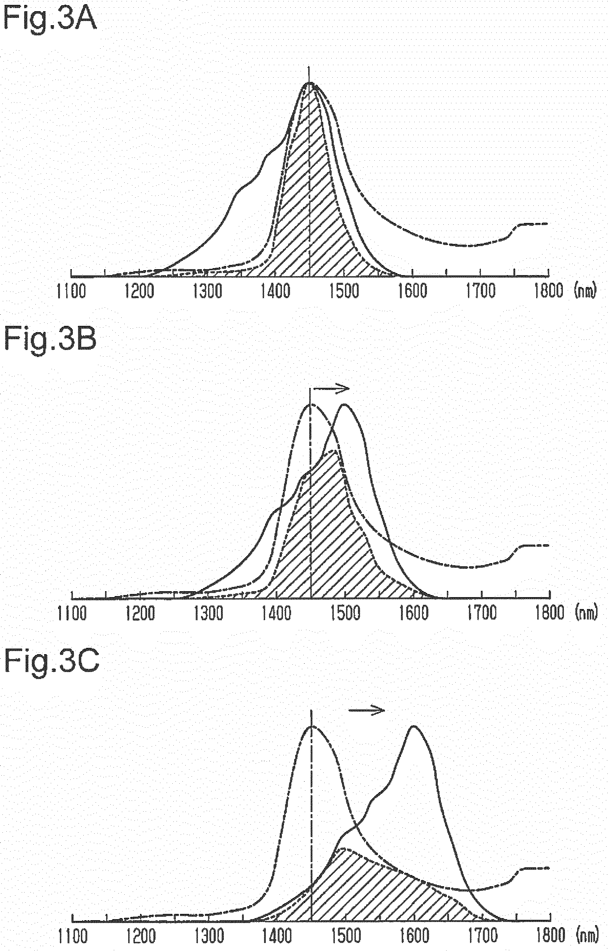

The output light of the body hair light emitting device 1 will now be described with reference to Figs. 3A to 3C. The controller 50 controls the spectrum of the output light of the body hair light emitting device 1 with the output of the light source 80.

In Figs. 3A to 3C, the single-dashed line represents the light absorption spectrum of water. The light absorption spectrum indicates the absorbance in the wavelength range from 1100 nm to 1800 nm as a value relative to the peak absorbance (reference absorbance).

In Figs. 3A to 3C, the solid line represents the spectrum of the output light of the body hair light emitting device 1 (hereinafter, "the output spectrum"). The output spectrum indicates the intensity in the wavelength range from 1100 nm to 1800 nm as a value relative to the peak intensity (reference intensity).

In Figs. 3A to 3C, the reference absorbance in the light absorption spectrum and the reference intensity in the output spectrum are each set to "1," and the two spectrums are indicated superposed with each other. The output spectrum in each diagram corresponds to a relative spectrum that uses the absorption spectrum as the reference spectrum.

In Figs. 3A to 3C, the light absorption spectrum and the output spectrum are indicated as spectrums obtained under a standard temperature environment. The standard temperature environment is, for example, a situation in which the atmosphere temperature is the room temperature.

The light absorption spectrum of water will now be described.

The light absorption spectrum is the light absorbance that has a significant level from approximately 1150 nm. In the wavelength range from 1150 nm to 1370 nm, as the wavelength becomes longer, the absorbance of the light absorption spectrum slightly increases. The light absorption spectrum has a light absorption peak region in the range from 1370 to 1540 nm. That is, the absorption peak, which indicates the maximum absorbance, exists in the wavelength range from 1100 nm to 1800 nm. The basal portion of the short-wavelength side of the absorption peak region exists at approximately 1370 nm, which is a shorter wavelength than the light absorption peak. The basal portion of the short-wavelength side has a convex inflection point. The basal portion of the long-wavelength side of the absorption peak region exists at approximately 1540 nm, which is a longer wavelength than the light absorption peak. The basal portion of the long-wavelength side has a concave inflection point. The light absorption peak exists at 1450 nm. The light absorption peak region has a convex inflection point at 1495 nm and a concave inflection point at approximately 1540 nm. The light absorption spectrum has another peak region in the wavelength range from 1750 nm to 1800 nm.

The output spectrum will now be described.

Fig. 3A illustrates the output spectrum when the temperature of the LED chip 81 (hereinafter, "the light source temperature") belongs to the reference temperature region (hereafter, the "reference output spectrum"). Fig. 3B illustrates the output spectrum when the light source temperature belongs to a first temperature region, which is higher than the reference temperature region. Fig. 3C illustrates the output spectrum when the light source temperature belongs to a second temperature region, which is higher than the first temperature region. The reference temperature corresponds to the light source temperature when the LED chip 81 is sufficiently releasing heat under a standard temperature environment.

The reference output spectrum includes a significant component in the wavelength range from 1200 nm to 1600 nm. The reference output spectrum does not include a significant component outside the wavelength range from 1200 nm to 1600 nm. A significant component refers to a component that may affect the promotion of the hair growth effect.

The reference output spectrum has a main peak region including the output light peak that indicates the maximum intensity in the wavelength range from 1200 nm to 1600 nm. The reference output spectrum does not include a significant peak region except for the main peak region in the wavelength range from 1200 nm to 1600 nm. That is, the reference output spectrum includes a single peak region in the wavelength range from 1200 nm to 1600 nm. The significant peak region is a peak region (spectrum) that may affect the promotion of the hair growth effect.

The main peak region includes an output light peak at, for example, 1450 nm, which substantially conforms to the wavelength of light absorption peak. The basal portion of the short-wavelength side of the main peak region exists at approximately 1200 nm, which is a shorter wavelength than the output light peak. Further, the basal portion of the long-wavelength side of the main peak region exists at approximately 1600 nm, which is a longer wavelength than the output light peak. The main peak region may include an output light peak at a wavelength other than 1450 nm, The preferred range of the output light peak in the main peak region may be, for example, the wavelength range from 1350 nm to 1550 nm.

In one example, the main peak region has a convex inflection point in the wavelength range from 1330 nm to 1360 nm. The main peak region has a concave inflection point in the wavelength range from 1370 nm to 1390 nm. The main peak region has a convex inflection point in the wavelength range from 1390 nm to 1410 nm. The main peak region has a concave inflection point in the wavelength range from 1410 nm to 1420 nm. The main peak region has a convex inflection point in the wavelength range from 1420 nm to 1430 nm. The main peak region has a concave inflection point in the wavelength range from 1430 nm to 1440 nm. The main peak region does not have a clear inflection point in the wavelength range from 1450 nm to 1600 nm. The intensity at the peak of the main peak region (output light peak) is, for example, 60 µW/cm²/nm.

The main peak has at least the four characteristics described below.

The first characteristic will now be described. The integral value of the peak region short-wavelength component, which is the main peak region component at the short-wavelength side of the peak (output light peak) of the main peak region, is larger than the integral value of the peak region long-wavelength component, which is the main peak region component at the long-wavelength side of the peak of the main peak region.

The peak region short-wavelength component includes components from the basal portion at the short-wavelength side of the main peak region to the peak of the main peak region. One example of an integral value of the peak region short-wavelength component is 5400 µW/cm². The peak region long-wavelength component includes components from the peak of the main peak region to the basal portion at the long-wavelength side of the main peak region. One example of the integral value of the peak region long-wavelength component is 4338 µW/cm².

The second characteristic will now be described. The short-wavelength side maximum gradient, which is the maximum gradient of the main peak region at the short-wavelength side of the peak (output light peak) of the main peak region, is smaller than the long-wavelength side maximum gradient, which is the maximum gradient of the main peak region-wavelength component at the long-wavelength side of the peak of the main peak region.

The short-wavelength side maximum gradient is located between the wavelength of the peak (output light peak) of the main peak region and a predetermined wavelength at the short-wavelength side of the peak of the main peak region. One example of the short-wavelength side maximum gradient is located in the wavelength range from 1420 nm to 1450 nm. One example of the short-wavelength side maximum gradient is 0.59 µW/cm²/nm².

The long-wavelength side maximum gradient is located between the wavelength of the peak (output light peak) of the main peak region and a predetermined wavelength at the long-wavelength side of the peak of the main peak region. One example of the long-wavelength side maximum gradient is located in the wavelength range from 1450 nm to 1510 nm. One example of the long-wavelength side maximum gradient is 1.1 pW/cm²/nm².

The third characteristic will now be described, The long-wavelength maximum gradient is larger than the long-wavelength side light absorbance gradient, which is the maximum gradient of the light absorption peak region at the long-wavelength side of the peak (light absorption peak) of the light absorption peak region of water. One example of the long-wavelength side light absorption gradient is located in the wavelength range from 1490 nm to 1530 nm.

The fourth characteristic will now be described. The short-wavelength maximum gradient is smaller than the short-wavelength side light absorbance gradient, which is the maximum gradient of the light absorption peak region at the short-wavelength side of the peak (light absorption peak) of the light absorption peak region of water. One example of the short-wavelength side light absorption gradient is located in the wavelength range from 1420 nm to 1450 nm.

The inventors of the present invention have studied the relationship between the emission time of light and the amount of light absorbed in a living body (hereinafter, "the light absorption amount"). Based on the findings obtained from the results of the study, the inventors have specified the output spectrum having the above four characteristics. The items studied by the inventors of the present invention will now be described in detail.

In the conventional body hair light emission device, the hair growth effect becomes difficult to promote as the light emission time becomes longer. It is understood that this is because the light absorption amount decreases. It is understood that the light absorption amount decreases for the reasons described below.

In the light output from the body hair light emitting device, the living body mainly absorbs components superposed with the light absorption spectrum of water. Further, in the light output from the body hair light emitting device, in particular, the living body has a tendency to absorb components superposed with the light absorption peak region of water. Thus, the light absorption amount is mainly dependent on the amount of light output from the body hair light emitting device and the amount of the portion superposed with the light absorption spectrum of water in the output light.

When the light source is temperature-dependent, a rise in the temperature of the light source causes an effect in which the spectrum of light shifts toward the long-wavelength side of the reference spectrum (hereinafter, "spectrum shift"). The amount of the spectrum shifted by the spectrum shift (hereinafter, "the shift amount"), increases as the temperature of the light source becomes higher. The temperature of the light source rises as the emission time of light becomes longer. Thus, a longer light emission time increases the shift amount

When the shift amount increases, the difference increases between the reference spectrum (light absorption spectrum) of the body hair light emitting device and the spectrum of the light output from the body hair light emitting device. Thus, in the amount of light output from the body hair light emitting device, the portion superposed with the light absorption spectrum of water decreases. A decrease in the superposed portion of the output light and the light absorption spectrum of water reduces the light absorption amount even when there is no change in the amount of light output from the body hair light emitting device. Thus, it becomes difficult to promote the hair growth effect.

The inventors of the present invention have studied the spectrum of light of which the portion superposed with the light absorption spectrum of water does not decrease much even when a spectrum shift increases the shift amount of the spectrum of light. The inventors have obtained the findings described below from the results of the study. When the integral value of the peak region short-wavelength component is larger than the integral value of the peak-region long-wavelength component, the light absorption amount does not decrease that easily even if a spectrum shift increases the shift amount of the spectrum of light compared to when the integral value of the peak region short-wavelength component is smaller than or equal to the integral value of the peak-region long-wavelength component. It is understood that this is due to the following reason.

A spectrum shift shifts the spectrum in one direction (long-wavelength side). Thus, an increase in the shift amount of the spectrum increases the portion of the peak region long-wavelength component that becomes non-superposed with the light absorption spectrum of water as compared with the peak region short-wavelength component. In contrast, the peak-region short-wavelength component exists in the short-wavelength side of the peak-region long-wavelength component. Thus, the portion that becomes non-superposed with the light absorption spectrum of water as the shift amount of the spectrum increases does not increase as much as the peak region long-wavelength portion. Accordingly, when the integral value of the peak-region short-wavelength component is larger than the integral value of the peak region long-wavelength component, the light absorption amount does not decrease much even when a spectrum shift occurs. Thus, even if the emission time of light increases, the effect for promoting hair growth does not decrease much.

The inventors of the present invention have checked the influence a spectrum shift has on the light absorption amount based on a comparison with the spectrum of light (hereinafter, "the comparative spectrum") output from a light emitting device of a comparative example.

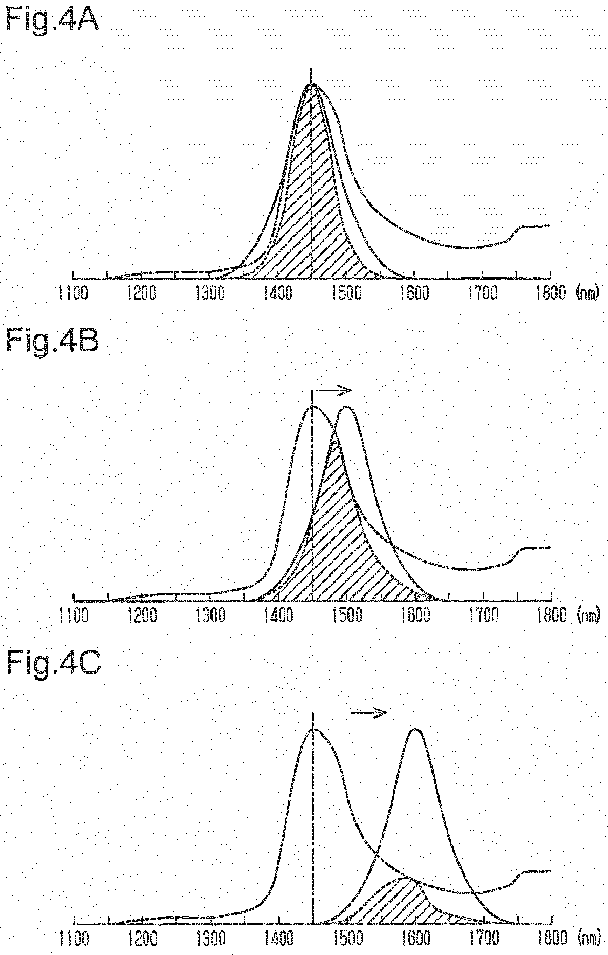

The comparative spectrum will now be described with reference to Figs. 4A to 4C.

In Figs. 4A to 4C, the single-dashed line represents the light absorption spectrum of water. The light absorption spectrum indicates the absorbance in the wavelength range from 1100 nm to 1800 nm as a value relative to the peak absorbance (reference absorbance).

In Figs. 4A to 4C, the solid line represents the spectrum of the light output from the light emitting device of the comparative example (hereinafter, "the comparative speotrum"). The comparative spectrum indicates the intensity in the wavelength range from 1100 nm to 1800 nm as a value relative to the peak intensity (reference intensity).

Figs. 4A to 4C illustrate the light absorption spectrum and the comparative spectrum obtained under a standard temperature environment. Fig. 4A illustrates the comparative spectrum when the light source temperature belongs to the reference temperature region (hereinafter, "the reference comparative spectrum"). Fig. 4B illustrates the comparative spectrum when the light source temperature belongs to the first temperature region. Fig. 4B illustrates the comparative spectrum when the light source temperature belongs to the second temperature region.

In Fig. 4A, the reference comparative spectrum has a significant component in the wavelength range from 1200 nm to 1600 nm. The reference comparative spectrum does not have a significant component outside the wavelength range from 1200 nm to 1600 nm.

The reference comparative spectrum has a comparative peak region including the maximum intensity as the output light peak in the wavelength range from 1200 nm to 1600 nm. The reference comparative spectrum does not have a significant peak region outside the comparative peak region in the wavelength range from 1200 nm to 1600 nm. That is the reference comparative spectrum has a single peak region in the wavelength range from 1200 nm to 1600 nm.

The comparative peak region includes a comparative peak region short-wavelength component, which is a component at the short-wavelength side of the peak (output light peak) of the comparative peak region, and a comparative peak region wavelength component, which is a component at the long-wavelength side of the peak of the comparative peak region. The comparative peak region is shaped to be in substantial line symmetry with respect to a symmetric line that extends perpendicular to the peak of the comparative peak region. That is, the comparative peak region short-wavelength component and the comparative peak region long-wavelength component are in a line symmetry relationship with respect to the symmetry line.

The comparative peak region has an output light peak at, for example, 1450 nm, which substantially coincides with the wavelength of the light absorption peak. The basal portion of the short-wavelength side of the comparative peak region exists at approximately 1300 nm, which is a shorter wavelength than the output light peak. Further, the basal portion of the long-wavelength side of the comparative peak region exists at approximately 1600 nm, which is a longer wavelength than the output light peak. The intensity of the peak (output light peak) of the comparative peak region is, for example, 60 µW/cm²/nm.

The spectrum shift of the comparative spectrum will now be described with reference to Figs, 4A to 4C.

When an operator operates an output operation unit of the light emitting device of the comparative example, a light source starts to output light When the emission time of the light output from the light source is shorter than a first predetermined time, the light source temperature belongs to the reference temperature region. Here, the comparative spectrum is as indicated by the reference comparative spectrum of Fig. 4A. When the emission time is longer than or equal to the first predetermined time, the light source temperature shifts to the first temperature region and causes a spectrum shift. Consequently, for example, the light source temperature rises and changes to the comparative spectrum illustrated in Fig. 4B. When the emission time is longer than or equal to a second predetermined time, which is longer than the first predetermined time, the light source temperature further rises and shifts to the second temperature region. Consequently, for example, the comparative spectrum illustrated in Fig. 4B changes to the comparative spectrum illustrated in Fig. 4C as the light source temperature rises.

During the process in which the reference comparative spectrum of Fig. 4A changes to the comparative spectrum of Fig, 4B, the portions superposed with the light absorption peak region of water decreases in the comparative peak region long-wavelength component and the comparative peak region short-wavelength component. Further, during the process in which the reference comparative spectrum of Fig. 4B changes to the comparative spectrum of Fig. 4C, the portions superposed with the light absorption peak region of water further decreases in the comparative peak region long-wavelength component and the comparative peak region short-wavelength component.

The hatched portions in Figs, 4A to 4C each illustrate the absorption amount of light in the light emitting device of the comparative example. The light absorption amount decreases in the order of the reference comparative spectrum of Fig. 4A, the comparative spectrum of Fig. 4B, and the comparative spectrum of Fig. 4C. That is, the light absorption amount decreases as the light emission time becomes longer. The light absorption amount at each wavelength of the comparative peak region may mainly be obtained from the product of the light intensity at each wavelength and the absorption of water.

The spectrum shift of the output spectrum will now be described with reference to Figs. 3A to 3C.

When an operator operates the output operation unit 42, the controller 50 starts the output of light from the light source 80. When the emission time of the light output from the light source 80 is less than a first predetermined time, the light source temperature belongs to the reference temperature region. Here, the output spectrum is as indicated by the reference output spectrum of Fig. 3A. When the emission time is longer than or equal to the first predetermined time, the light source temperature shifts to the first temperature region and causes a spectrum shift. Consequently, for example, the light source temperature rises and changes to the output spectrum illustrated in Fig. 3B. When the emission time is longer than or equal to a second predetermined time, the light source temperature further rises and shifts to the second temperature region. Consequently, for example, the comparative spectrum illustrated in Fig. 3B changes to the output spectrum illustrated in Fig. 3C as the light source temperature rises.

During the process in which the reference output spectrum of Fig. 3A changes to the output spectrum of Fig. 3B, the portion superposed with the light absorption peak region of water decreases in the peak region long-wavelength component. Further, during the process in which the reference output spectrum of Fig. 3A changes to the output spectrum of Fig. 3B, the portion superposed with the light absorption peak region of water subtly decreases in the peak region short-wavelength component. Thus, in contrast with the comparative spectrum, the output spectrum subtly decreases the amount of light absorbed by a living body even when the emission time of light increases.

During the process in which the output spectrum of Fig. 3B changes to the output spectrum of Fig. 3C, the portion superposed with the light absorption peak region of water further decreases in the peak region long-wavelength component. During the process in which the reference output spectrum of Fig. 3B changes to the output spectrum of Fig. 3C, the portion superposed with the light absorption peak region of water also decreases in the short-wavelength component. However, the area of the peak region short-wavelength component is larger than the short-wavelength component of the comparative peak region. Thus, the portion superposed with the light absorption spectrum of water does not decrease much in the entire main peak region as compared with the comparative peak region. Accordingly, in comparison with the comparative spectrum, the output spectrum does not decrease the amount of fight absorbed by the living body that much even when the emission time of light increases.

The hatched portions in Figs. 3A to 3C each illustrate the absorption amount of the light output from the body hair light emitting device 1. In one example, the light absorption amount is generally the same in the reference output spectrum of Fig. 3A and the output spectrum of Fig. 3B. The light absorption amount of the output spectrum of Fig. 3B is less than the light absorption amount of the reference output spectrum of Fig. 3A and light absorption amount of the output spectrum of Fig. 3B. The light absorption amount at each wavelength in the main peak region may mainly be obtained from the product of the light intensity at each wavelength and the absorption of water.

The body hair light emitting device has the advantages described below,
(1) In the main peak region of the output light, the integral value of the peak region short-wavelength component is larger than the integral value of the peak region long-wavelength component. Thus, when a spectrum shift occurs, the superposed portion of the main peak region and the light absorption peak region of water does not decrease much. Accordingly, a decrease in the light absorption amount can be limited even when the emission time of light is increased.
(2) In the main peak region of the output light, the short-wavelength side maximum gradient is smaller than the long-wavelength side absorbance gradient. Thus, even when a spectrum shift occurs, the superposed portion of the main peak region and the light absorption peak region of water does not decrease much. This further improves the effect described in advantage (1).
(3) The long-wavelength side maximum gradient of the main peak region of the output light is larger than the long-wavelength side light absorption gradient of the light absorption peak region. Thus, even when a spectrum shift occurs, the portion of the peak region long-wavelength component that is not superposed with the light absorption peak region of water does not decrease much. This further improves the effect described in advantage (1).
(4) The short-wavelength side maximum gradient of the main peak region of the output light is smaller than the long-wavelength side light absorption gradient of the light absorption peak region. Thus, even when a spectrum shift occurs, the superposed portion of the peak region short-wavelength component and the light absorption peak region of water does not decrease much. This further improves the effect described in advantage (1).
(5) The body hair light emitting device 1 is advantageous over the light emitting device of the comparative example in the points described below. Further, the output spectrum (comparative spectrum) of the light output from the light emitting device of the comparative example differs from the output spectrum of the body hair light emitting device 1 in the points described below.

The comparative spectrum has a peak region (comparative peak region) that includes the maximum intensity as the output light peak in the wavelength range from 1200 nm to 1600 nm. The comparative peak region substantially has a line symmetry relationship with respect to a symmetric line that extends perpendicular to the peak (output light peak) of the comparative peak region.

The comparative peak region includes the comparative peak region short-wavelength component at the short-wavelength side of the peak and the comparative peak region long-wavelength component at the long-wavelength side of the peak. In one example, the comparative peak region short-wavelength component may have the same shape as the peak region short-wavelength component in the main peak region of the body hair light emitting device 1, However, the comparative peak region long-wavelength component has a line symmetry relationship with the comparative peak region short-wavelength component. Thus, the integral value of the comparative peak region long-wavelength component is larger than the integral value of the peak region long-wavelength component of the body hair light emitting device 1 in the above embodiment. Thus, in comparison with the peak region long-wavelength component of the body hair light emitting device 1, the portion of the comparative peak region long-wavelength component superposed with the light absorption peak region may be large.

The comparative peak region long-wavelength component exists in the shift direction side of the spectrum shift from the peak of the light absorption peak region. Thus, when a spectrum shift occurs, the ratio of the comparative peak region long-wavelength component that contributes to the formation of the portion superposed with the light absorption peak region is smaller than that of the comparative peak region short-wavelength component. That is, when a spectrum shift occurs, the portion of the comparative peak region long-wavelength component superposed with the light absorption peak region is formed at a lower efficiency than the comparative peak region short-wavelength component.

The integral value of the entire comparative spectrum in the light emitting device of the comparative example is larger than the integral value of the entire output spectrum of the body hair light emitting device 1. Accordingly, the light emitting device of the comparative example uses a large amount of electric energy to output light Thus, the light source of the light emitting device of the comparative example tends to become hotter than the light source 80 of the body hair light emitting device 1. Accordingly, spectrum shifts easily occur in the light emitting device of the comparative example.

In this manner, in the light emitting device of the comparative example, the integral value of the comparative peak region long-wavelength component is large. This easily obtains a portion superposed with the light absorption peak region and easily promotes spectrum shifts as the consumed energy increases. Thus, when a spectrum shift occurs, the efficiency for forming the superposed portion of the comparative peak region and the light absorption peak region of water is likely to decrease.

In contrast, with the body hair light emitting device 1, the integral value of the peak region long-wavelength component is smaller than the integral value of the peak-region short-wavelength component. Thus, from the aspect of obtaining a superposed portion of the main peak region and the light absorption peak region of water, the next two advantages are obtained over the light emitting device of the comparative example.

The first advantage will now be described. In the main peak region, the peak region long-wavelength component, which contributes less than the peak region short-wavelength component to the formation of the superposed portion, has a low integral value. This reduces the energy consumed to obtain the superposed portion of the main peak region and the light absorption peak region of water compared to the light emitting device of the comparative example,

The second advantage will now be described. The integral value of the peak region long-wavelength component in the main peak region is smaller than the integral value of the comparative peak region long wavelength component. Thus, the electric energy used to obtain the superposed portion of the main peak region and the light absorption of water is small compared to the light emitting device of the comparative example

The body hair light emitting device 1 may be implemented in other embodiments that differ from the above embodiment. Other embodiments include, for example, the modified examples described below. The modified examples may be combined with one another as long as there are no technical contradictions,

The power supply 30 may convert AC current of a commercial power supply to DC current and supply the converted power to each electric block.

The operation unit 40 may have the form of, for example, a switch or a touch panel.

The LED lamp may have a bullet-type form.

The output light may be specified by the following characteristics. The main peak region of the output light includes a short-wavelength side base point, which is the base point of the main peak region that exists at the short-wavelength side of the peak of the main peak region, and a long-wavelength side base point, which is the base point of the main peak region that exists at the long-wavelength side of the peak of the main peak region. The main peak region has a gradient between two points at the short-wavelength side specified by the short-wavelength side base point and the peak of the main peak region. Further, the main peak region has a gradient between two points at the long-wavelength side specified by the peak of the main peak region and the long-wavelength side base point. The gradient between the two points at the short-wavelength side is smaller than the gradient between the two points at the long-wavelength side.

When the light source temperature belongs to one of the reference temperature region, the first temperature region, the second temperature region, and a further temperature region, the peak of the main peak region preferably exists in the wavelength range from 1350 nm to 1550 nm and, more preferably, exists in the wavelength range from 1400 nm to 1500 nm.

When the light source temperature belongs to the main temperature region, the peak of the main peak region preferably exists in the wavelength range from 1350 nm to 1550 nm and, more preferably, exists in the wavelength range from 1400 nm to 1500 nm. The main temperature region is a temperature region in which the time to which the light source temperature belongs is the longest during the period from which the use of the body hair light emitting device 1 starts to when the use ends.

For example, during the period from which the use of the body hair light emitting device 1 starts to when the use ends, it is assumed that the light source temperature shifts in the order of the reference temperature region, the first temperature region, and the second temperature region. In this case, when the time in which the light source temperature belongs to the first temperature region is longer than the time in which the light source temperature belongs to the reference temperature region and the second temperature region, the first temperature region corresponds to the main temperature region.

The main temperature region may be set in advance based on the usage conditions set for the body hair light emitting device 1. When the main temperature region differs in accordance with the specified time selected in accordance with the operation of the emission time setter 43, for example, one of a plurality of specified times is included in the usage condition of the body hair light emitting device 1. The main temperature region may be specified based on the usage condition.

The above modified example specifies the preferred wavelength range for the peak of the main peak region based on the relationship with the main temperature region. This modified example may further have one of structures (a) and (b) that are described below.
(a) The difference between the integral value of the peak region short-wavelength component and the integral value of the peak region long-wavelength component is smaller than the difference between the integral value of the peak region short-wavelength component and the integral value of the peak region long-wavelength component in the output light spectrum of the above embodiment. That is, the shape of the entire main peak region is further symmetric with respect to a symmetry line extending perpendicular to the peak of the main peak region compared to the shape of the entire main peak region of the above embodiment.
(b) The difference between the short-wavelength side maximum gradient and the long-wavelength side maximum gradient is smaller than the short-wavelength side maximum gradient and the long-wavelength side maximum gradient in the output light spectrum of the above embodiment. That is, the shape of the entire main peak region is further symmetric with respect to a symmetry line extending perpendicular to the peak of the main peak region compared to the shape of the entire main peak region of the above embodiment.

### [Example]

The inventors of the present embodiment have conducted a hair growth verification test, which is described below to check the difference in the hair growth promotion effect obtained by the body hair light emitting device of the above embodiment and the hair growth promotion effect obtained by the light emitting device of a comparative example. The light output by the light emitting device of the comparative example has the comparative spectrum illustrated in Figs. 4A to 4C.

In the hair growth verification test, the lower limb of an adult male was used as an emission subject portion of light. In the hair growth verification test, the number of samples of the emission subject section was set to four. In the hair growth verification test, part of the emission subject portion was set as an observation subject portion. In the hair growth verification test, a predetermined part of the emission subject portion was entirely shaved to remove body hair and set as the observation subject portion. The observation subject portion has an area of 4 cm x 4 cm.

In the hair growth verification test, various test conditions were specified, and light was emitted to the observation subject portion under the specified test conditions. In the hair growth test, emission dates, emission period, output frequency, and observation timing were specified as described below.

Five days were set as the emission dates. On each of the five days of the emission dates, light was emitted once at a specified emission time and output frequency.

Three emission periods were set, 5 minutes, 10 minutes, and 20 minutes. The emission period refers to the period during which the body hair light emitting device 1 emits light each day to the observation subject portion.

Three output frequencies were set, 0 Hz (continuous emission), 100 Hz, and 500 Hz. The output frequency was the output frequency TL of the light emitted to the emission subject portion.

Four observation timings were set, prior to start, 10 days, 20 days, and 30 days. The day following the day that light was first emitted to the emission subject portion was set as the first day.

Three items were observed at each observation timing to evaluate the growth of hair in the emission subject portion. The three items are the amount of hair, the thickness of hair, and the length of hair. The thickness of hair refers to the diameter at the basal portion of a single hair. The basal portion of a single hair refers to the portion of the hair growing from the skin that is closest to the skin.

The inventors of the present invention set the test conditions of the hair growth verification test by combining the emission period, the output frequency, and the observation timing. Tests were conducted on an example and the comparative example under the same test conditions. The hair growth verification test results of the example are illustrated in "Table 1." The hair growth verification test results of the comparative example are illustrated in "Table 2."

**[Table 1]**

| Hair Growth Verification Test Results of Example | | | | | | | | | | | | | |
|---|---|---|---|---|---|---|---|---|---|---|---|---|---|
| Emission Time (min) | Output Frequency (Hz) | Amount of Hair (Quantity) | | | | Hair Thickness (mm) | | | | Hair Lunght (mm) | | | |
| | | Prior to start | 10 Days | 20 Days | 30 Days | Prior to start | 10 Days | 20 Days | 30 Days | Prior to start | 10 Days | 20 Days | 30 Days |
| 20 | 500 | 0 | 15 | 45 | 60 | 0.00 | 0.01 | 0.02 | 0.03 | 0.0 | 1.0 | 2.0 | 4.0 |
| | 100 | 0 | 12 | 48 | 58 | 0.00 | 0.01 | 0.01 | 0.03 | 0.0 | 0.8 | 2.2 | 3.7 |
| | Continuous Emission | 0 | 13 | 45 | 57 | 0.00 | 0.01 | 0.02 | 0.02 | 0.0 | 1.1 | 1.8 | 3.5 |
| 10 | 500 | 0 | 8 | 23 | 30 | 0.00 | 0.01 | 0.01 | 0.02 | 0.0 | 0.5 | 1.0 | 2.0 |
| | 100 | 0 | 6 | 22 | 29 | 0.00 | 0.01 | 0.01 | 0.02 | 0.0 | 0.4 | 1.1 | 1.9 |
| | Continuous Emission | 0 | 7 | 23 | 29 | 0.00 | 0.01 | 0.01 | 0.01 | 0.0 | 0.6 | 0.9 | 1.8 |
| 5 | 500 | 0 | 4 | 11 | 15 | 0.00 | 0.00 | 0.01 | 0.01 | 0.0 | 0.3 | 0.5 | 1.0 |
| | 100 | 0 | 3 | 11 | 15 | 0.00 | 0.00 | 0.00 | 0.01 | 0.0 | 0.2 | 0.6 | 0.9 |
| | Continuous Emission | 0 | 3 | 11 | 14 | 0.00 | 0.00 | 0.01 | 0.01 | 0.0 | 0.3 | 0.5 | 0.9 |

**[Table 2]**

| Hair Growth Verification Test Results of Comparative Example | | | | | | | | | | | | | |
|---|---|---|---|---|---|---|---|---|---|---|---|---|---|
| Emission Time (min) | Output Frequency (Hz) | Amount of Hair (Quantity) | | | | Hair Thickness (mm) | | | | Hair Length (mm) | | | |
| | | Prior to Start | 10 Days | 20 Days | 30 Days | Prior to Start | 10 Days | 20B | 30B | Prior to Start | 10 Days | 20 Days | 30 Days |
| 20 | 500 | 0 | 8 | 23 | 30 | 0.00 | 0.01 | 0.01 | 0.00 | 0.0 | 0.5 | 1.0 | 2.0 |
| | 100 | 0 | 6 | 22 | 29 | 0.00 | 0.01 | 0.01 | 0.02 | 0.0 | 0.1 | 1.1 | 1.9 |
| | Continuous Emission | 0 | 7 | 23 | 29 | 0.00 | 0.01 | 0.01 | 0.01 | 0.0 | 0.6 | 0.9 | 1.8 |
| 10 | 500 | 0 | 4 | 11 | 15 | 0.00 | 0.00 | 0.01 | 0.01 | 0.0 | 0.3 | 0.5 | 1.0 |
| | 100 | 0 | 3 | 11 | 14 | 0.00 | 0.00 | 0.00 | 0.01 | 0.0 | 0.2 | 0.6 | 0.9 |
| | Continuous Emission | 0 | 3 | 11 | 14 | 0.00 | 0.00 | 0.01 | 0.01 | 0.0 | 0.3 | 0.5 | 0.9 |
| 5 | 500 | 0 | 2 | 6 | 8 | 0.00 | 0.00 | 0.00 | 0.00 | 0.0 | 0.1 | 0.3 | 0.5 |
| | 100 | 0 | 2 | 5 | 7 | 0.00 | 0.00 | 0.00 | 0.00 | 0.0 | 0.1 | 0.3 | 0.5 |
| | Continuous Emission | 0 | 2 | 6 | 7 | 0.00 | 0.00 | 0.00 | 0.00 | 0.0 | 0.1 | 0.2 | 0.4 |

"Table 1" and "Table 2" indicate the following facts.

The amount of hair in the example was greater than the amount of hair in the comparative example The amount of hair was greatest under the conditions of emission time: 20 minutes, output frequency: 500 Hz, and observation timing: 30 days.

The thickness of hair in the example was greater than the thickness of hair in the comparative example. The thickness of hair was greatest under the conditions of emission time: 20 minutes, output frequency: 500 Hz, and observation timing: 30 days.

The length of hair in the example was greater than the length of hair in the comparative example. The length of hair was greatest under the conditions of emission time. 20 minutes, output frequency: 500 Hz, and observation timing: 30 days.

The above measurement results indicate that even when the emission time of light increases, the decrease in the light absorption amount based on the output light of the body hair light emitting device 1 is smaller than that of the light absorption amount based on the output light of the light emitting device of the comparative example. This indicates that the use of the body hair light emitting device promotes the hair growth effect more easily than when using the light emitting device of the comparative example.

## Claims

1. A body hair light emitting device configured to emit light, the body hair light emitting device comprising:
a light source configured to output light; and
a controller connected to the light source, wherein the controller is configured to control output of the light source so that output light of the body hair light emitting device resulting from the output of the light source has a main peak region including an output light peak, which indicates maximum intensity, in a wavelength range from 1350 nm to 1550 nm and so that an integral value of a peak region short-wavelength component, which is the main peak region component at the short-wavelength side of the output light peak, is larger than an integral value of a peak region long-wavelength component, which is the main peak region component at the long-wavelength side of the output light peak.

2. The body hair light emitting device according to claim 1, wherein a short-wavelength side maximum gradient, which is the maximum gradient of the main peak region at the short-wavelength side of the output light peak, is smaller than a long-wavelength side maximum gradient, which is the maximum gradient of the main peak region at the long-wavelength side of the output light peak.

3. The body hair light emitting device according to claim 2, wherein
the light absorption spectrum of water has a light absorption peak region including a light absorption peak, which indicates maximum absorbance, in a wavelength range from 1440 nm to 1460 nm; and
the controller is configured to control the spectrum of the output light relative to the light absorption spectrum of water so that the long-wavelength side maximum gradient of the main peak region is larger than a long-wavelength side light absorbance gradient, which is the maximum gradient of the light absorption peak region at the long-wavelength side of the light absorption peak.

4. The body hair light emitting device according to claim 2 or 3, wherein
the light absorption spectrum of water has a light absorption peak region including a light absorption peak, which indicates maximum absorbance, in a wavelength range from 1440 nm to 1460 nm; and
the controller is configured to control spectrum of the output light relative to the light absorption spectrum of water so that the short-wavelength side maximum gradient of the main peak region is smaller than a short-wavelength side light absorbance gradient, which is the maximum gradient of the light absorption peak region at the short-wavelength side of the light absorption peak.

## Patentansprüche

1. Lichtemittierende Vorrichtung für Körperhaare, die konfiguriert ist, um Licht zu emittieren, wobei die lichtemittierende Vorrichtung für Körperhaare umfasst:
eine Lichtquelle, die konfiguriert ist, um Licht auszugeben; und
eine mit der Lichtquelle verbundene Steuerungseinheit, wobei die Steuerungseinheit konfiguriert ist, um die Ausgabe der Lichtquelle so zu steuern, dass das ausgegebene Licht der lichtemittierenden Vorrichtung für Körperhaare, das das Ergebnis der Ausgabe der Lichtquelle ist, einen Spitzenbereich mit einer ausgegebenen Lichtspitze hat, die eine maximale Intensität in einem Wellenlängenbereich von 1350 nm bis 1550 nm anzeigt, und so, dass ein Integralwert einer Komponente mit kurzer Wellenlänge im Spitzenbereich, die die Hauptspitzenbereichskomponente an der Seite der kurzen Wellenlänge der ausgegebenen Lichtspitze ist, größer ist als ein Integralwert einer Komponente mit langer Wellenlänge im Spitzenbereich, die die Hauptspitzenbereichskomponente an der Seite der langen Wellenlänge der ausgegebenen Lichtspitze ist.

2. Lichtemittierende Vorrichtung für Körperhaare nach Anspruch 1, wobei ein maximaler Gradient an der Seite der kurzen Wellenlänge, der der maximale Gradient des Hauptspitzenbereiches an der Seite der kurzen Wellenlänge der ausgegebenen Lichtspitze ist, kleiner ist als ein maximaler Gradient an der Seite der langen Wellenlänge, der der maximale Gradient des Hauptspitzenbereiches an der Seite der langen Wellenlänge der ausgegebenen Lichtspitze ist.

3. Lichtemittierende Vorrichtung für Körperhaare nach Anspruch 2, wobei
das Lichtabsorptionsspektrum von Wasser einen Lichtabsorptionsspitzenbereich mit einer Lichtabsorptionsspitze hat, die ein maximales Absorptionsvermögen in einem Wellenlängenbereich von 1440 nm bis 1460 nm anzeigt; und
die Steuerungseinheit konfiguriert ist, um das Spektrum des ausgegebenen Lichts relativ zum Lichtabsorptionsspektrum von Wasser so zu steuern, dass der maximale Gradient an der Seite der langen Wellenlänge des Hauptspitzenbereiches größer ist als ein Lichtabsorptionsgradient an der Seite der langen Wellenlänge, der der maximale Gradient des Lichtabsorptionsspitzenbereiches an der Seite der langen Wellenlänge der Lichtabsorptionsspitze ist.

4. Lichtemittierende Vorrichtung für Körperhaare nach Anspruch 2 oder 3, wobei das Lichtabsorptionsspektrum von Wasser einen Lichtabsorptionsspitzenbereich mit einer Lichtabsorptionsspitze hat, die ein maximales Absorptionsvermögen in einem Wellenlängenbereich von 1440 nm bis 1460 nm anzeigt; und
die Steuerungseinheit konfiguriert ist, um das Spektrum des ausgegebenen Lichts relativ zum Lichtabsorptionsspektrum von Wasser so zu steuern, dass der maximale Gradient an der Seite der kurzen Wellenlänge des Hauptspitzenbereiches kleiner ist als der Lichtabsorptionsgradient an der Seite der kurzen Wellenlänge, der der maximale Gradient des Lichtabsorptionsspitzenbereiches an der Seite der kurzen Wellenlänge der Lichtabsorptionsspitze ist.

## Revendications

1. Dispositif d'émission de lumière sur les poils configuré pour émettre de la lumière, le dispositif d'émission de lumière sur les poils comprenant :
une source de lumière configurée pour émettre de la lumière ; et
un dispositif de commande relié à la source de lumière, le dispositif de commande étant configuré pour commander l'émission de source de lumière afin que la lumière émise depuis le dispositif d'émission de lumière sur les poils résultant de l'émission de la source de lumière présente une région pic principale incluant un pic de lumière d'émission, qui indique l'intensité maximale, dans une plage de longueurs d'ondes de 1 350 nm à 1 550 nm et de sorte qu'une valeur intégrale d'un composant de courte longueur d'onde de région pic, qui est le composant de région pic principal sur le côté des longueurs d'ondes courtes du pic de lumière d'émission, soit supérieure à la valeur intégrale d'un composant de longueur d'onde longue de région pic, qui est le composant de région pic principal du côté des longueurs d'ondes longues du pic de lumière d'émission.

2. Dispositif d'émission de lumière sur les poils selon la revendication 1, un gradient maximal du côté des courtes longueurs d'ondes, qui est le gradient maximal de la région pic principale du côté des courtes longueurs d'ondes du pic de lumière d'émission, est inférieur au gradient maximal du côté des longueurs d'ondes longues, qui est le gradient maximal de la région pic principale du côté des longueurs d'ondes longues du pic de lumière d'émission.

3. Dispositif d'émission de lumière sur les poils selon la revendication 2,
le spectre d'absorption de lumière de l'eau présentant une région pic d'absorption de lumière incluant un pic d'absorption de lumière, qui indique l'absorbance maximale, dans une plage de longueurs d'ondes de 1 440 nm à 1 480 nm ; et
le dispositif de commande est configuré pour commander le spectre de lumière d'émission par rapport au spectre d'absorption de lumière de l'eau de sorte que le gradient maximal du côté des longueurs d'ondes longues de la région pic principale soit plus large qu'un gradient d'absorbance de lumière du côté des longueurs d'ondes longues, qui est le gradient maximal de la région pic d'absorption de lumière du côté des longueurs d'ondes longues du pic d'absorption de lumière.

4. Dispositif d'émission de lumière sur les poils selon la revendication 2 ou 3,
le spectre d'absorption de lumière de l'eau présentant une région pic d'absorption de lumière incluant un pic d'absorption de lumière, qui indique l'absorbance maximale, dans une plage de longueurs d'ondes de 1 440 nm à 1 460 nm ; et
le dispositif de commande étant configuré pour commander le spectre de la lumière d'émission par rapport au spectre d'absorption de lumière de l'eau de sorte que le gradient maximal du côté des courtes longueurs d'ondes de la région pic principale soit plus petit qu'un gradient d'absorbance de lumière du côté des courtes longueurs d'ondes, qui est le gradient maximal de la région pic d'absorption de lumière du côté des courtes longueurs d'ondes du pic d'absorption de lumière.
